# EUROPEAN PATENT APPLICATION

(11) **EP 2 487 212 A1**
(43) Date of publication of application: **15.08.2012**
(21) Application number: 10822090.6
(22) Date of filing: 07.10.2010
(51) Int. Cl.: C09D 175/04, C08G 18/10, C09D 175/08

(54) **COATING MATERIAL FOR HAND APPLICATION**

(30) Priority: 09.10.2009 JP 2009235613
(71) Applicant: Dyflex Corporation, Shinjuku-ku, Tokyo 163-0825 (JP)
(72) Inventor: TANIHARA Daisuke, Funabashi-shi Chiba 273-0027 (JP); YANO Noriyoshi, Funabashi-shi Chiba 273-0027 (JP)
(74) Representative: Sika Patent Attorneys
(86) International application number: PCT/JP2010/067650
(87) International publication number: WO 2011/043420

(57) **Abstract**

A coating agent for hand painting application obtained by a urethane resin composition obtained by allowing a main agent (A) and a curing agent (B) to react. The main agent (A) containing: an isocyanate group-terminated prepolymer (b) having diphenylmethane diisocyanate bound to the termini of a polyol component (a) containing a long chain polyol (e) which contains at least a polyhydroxy functional product such as a trihydroxy or higher-hydroxy functional product and having a molecular weight of 200 or greater; and unbound diphenylmethane diisocyanate(c). The curing agent (B) containing: a crosslinking agent (g) obtained by a short chain polyol (f) having a molecular weight of less than 200; and a long chain polyol (h) having a molecular weight of 200 or greater.

## Description

### Technical Field

The present invention relates to a coating agent for hand painting application containing a urethane resin composition which is used in waterproofing applications for buildings, and the like.
Priority is claimed on Japanese Patent Application No. 2009-235613, filed on October 9, 2009, the content of which is in incorporated herein by reference.

### Background Art

Urethane resin compositions used in the waterproofing applications for rooftops, verandas, corridors and the like of buildings, are classified into compositions for hand painting application which are applied using a trowel, a spatula or the like, and compositions for spray application which are applied by spraying.
Since urethane resin compositions for hand painting application require sufficient usable time, tolylene diisocyanate (TDI) is usually used as an isocyanate component. More specifically, a main agent containing an isocyanate group-terminated prepolymer obtained by TDI and a polyol (polyether polyol or the like), and a curing agent are used in a mixture.
As the curing agent, a curing agent containing a crosslinking agent obtained by using an amine compound such as diethyltoluenediamine (DETDA), 3,3'-dichloro-4,4'-diaminodiphenylmethane (MOCA) or a modified MOCA, is widely used.
At the time of production of the isocyanate group-terminated prepolymer, TDI and a polyol compound are reacted so as to prevent any monomer (TDI that is not involved in bonding) from remaining behind, in consideration of the harmfulness of TDI.

In urethane resin compositions for spray application, diphenylmethane diisocyanate (MDI) which is relatively highly safe is usually used as an isocyanate component. More specifically, a main agent containing an isocyanate group-terminated prepolymer obtained by using MDI and a polyol, and a curing agent are used in a mixture.
At the time of application, a spraying apparatus having a spray nozzle is used, and the main agent and the curing agent are mixed in a mixing apparatus provided at the spray nozzle, so that the mixture is sprayed through the spray nozzle in the form of mist.
Since MDI is used as the isocyanate component, the usable time is shortened. However, in the spray application, since the mixed liquid is applied immediately after mixing, the component does not cause any problems.

### Prior Art Document

### Patent Document

Patent Document 1: Japanese Unexamined Patent Application, First Publication No. 2007-284520

### Disclosure of the Invention

### Problem solved by the Present Invention

Because spray application has a problem with the scattering of the resin composition, hand painting application where scattering does not occur is frequently employed.
However, the urethane resin composition for hand painting application uses TDI as the isocyanate component to secure a usable time, and therefore, safety needs to be considered.
Investigations have been made regarding the use of MDI which is relatively highly safe, but because the curing rate is increased, it has been difficult to employ MDI in hand painting applications.
Accordingly, a coating agent for a hand painting application that does not use TDI has been demanded.
Furthermore, examples of the crosslinking agent used in the curing agent include amines compounds such as DETDA, MOCA and modified MOCA.
Among these, in the case of using DETDA, when MDI is used as an isocyanate component, it becomes difficult to secure the use time required for a hand painting application. Thus, TDI must be used. Furthermore, MOCA and modified MOCA can decrease the curing rate, and are thus advantageous in terms of use time. However, from the viewpoint of safety, there is a demand for a substitute.
The present invention was made in view of such circumstances, and it is an object according to the present to provide a coating agent for a hand painting application which can secure sufficient use time and is highly safe.

### Means for Solving the Problem

The coating agent for hand painting application according to the present invention is a coating agent for hand painting application containing a urethane resin composition that is obtainable by reacting a main agent (A) and a curing agent (B).
The main agent (A) is a main agent containing:
an isocyanate group-terminated prepolymer (b) having a diphenylmethane diisocyanate bonded to the termini of a polyol component (a) containing a long chain polyol (e) which contains at least a polyhydroxy functional product such as a trihydroxy or higher-hydroxy functional product and having a molecular weight of 200 or greater; and
unbound diphenylmethane diisocyanate (c).
The curing agent (B) contains a crosslinking agent (g) contains a short chain polyol (f) having a molecular weight of less than 200 and a long chain polyol (h) having a molecular weight of 200 or greater.
In regard to the curing agent (B), the mixing ratio of the OH group of the crosslinking agent (g) with respect to the total amount of OH groups of the crosslinking agent (g) and the long chain polyol (h), is preferably 60 to 90% on a molar basis.
The main agent (A) is preferably a product obtained by mixing diphenylmethane diisocyanate and the polyol component (a) so as to obtain a content of NCO groups of 4 to 9% by mass.
In the main agent (A), the polyol component (a) may contain a short chain polyol (d) having a molecular weight of less than 200.
In the main agent (A), the long chain polyol (e) may be a mixture of a dihydroxy functional product and a trihydroxy functional product.
In the curing agent (B), a mixture of any one of a polymeric polyol and a castor oil polyol, and a polyether polyol can be used as the long chain polyol (h).
In the curing agent (B), a polyether polyol can be used as the long chain polyol (h).
The main agent according to the present invention is a main agent (A) of the coating agent for hand painting application, the main agent (A) containing:
an isocyanate group-terminated prepolymer (b) having diphenylmethane diisocyanate bound to the termini of a polyol component (a) containing a long chain polyol (e) which contains at least a polyhydroxy functional product such as a trihydroxy or higher-hydroxy functional product and has a molecular weight of 200 or greater; and
unbound diphenylmethane diisocyanate (c).
The curing agent according to the present invention is a curing agent (B) of the coating agent for hand painting application, the curing agent (B) comprising:
a crosslinking agent (g) obtained by a short chain polyol (f) having a molecular weight of less than 200, and
a long chain polyol (h) having a molecular weight of 200 or greater.

### Effects Obtained by the Present Invention

The coating agent for hand painting application according to the present can form a waterproof coating film (urethane waterproof material) when applied to rooftops, verandas, corridors and the like of buildings and cured.
Since the curing rate can be suppressed, a sufficient usable time can be secured, and thus a hand painting application can be achieved by using a tool such as a trowel, a spatula, a rake, or a squeegee.
Furthermore, since MDI which is less harmful to the human body is used, the coating agent for a hand painting application is excellent in terms of safety.

### Best Mode for Carrying Out the Invention

Hereinafter, the present invention will be described in detail.
The coating agent for hand painting application according to the present is a coating agent containing a urethane resin composition obtainable by reacting at least a main agent (A) and a curing agent (B) shown below:

### (1) Main agent (A)

The main agent (A) contains an isocyanate group-terminated prepolymer (b) having diphenylmethane diisocyanate (hereinafter, referred to as MDI) bonded to the termini of a polyol component (a), and an unbound MDI (c) (hereinafter, referred to as surplus MDI (c)).
The polyol component (a) contains a long chain polyol (e). Here, the polyol is a compound having a plurality of hydroxyl groups.

The long chain polyol (e) is a polyol having a molecular weight (average molecular weight) of 200 or greater, and for example, one or two or more of a polyether polyol, a polyester polyol and a polycarbonate polyol can be used.
Examples of the polyether polyol include polyethylene glycol, polypropylene glycol, and polytetramethylene glycol.
Examples of the polyester polyol include polyethylene adipate diol, polybutylene adipate diol, polyethylene succinate diol, and polybutylene succinate diol.
Examples of the polycarbonate polyol include polytetramethylene carbonate diol, and polyhexamethylene carbonate diol.
Furthermore, a polymeric polyol or a plant-derived polyol (castor oil, linseed oil, or the like) can also be used. The polymeric polyol is a product obtainable by, for example, polymerizing a monomer such as styrene or acrylonitrile, in a reaction solvent such as polypropylene glycol.
As the long chain polyol (e), these compounds may be used individually, or a mixture of two or more compounds may be used.
According to the invention, the number average molecular weight or the mass average molecular weight can be used as the average molecular weight.

The long chain polyol (e) contains at least a polyhydroxy functional product (polyfunctional product) such as a trihydroxy functional product (trifunctional product) or a higher-hydroxy functional product.
The polyhydroxy functional product may be a product having 3 or more hydroxyl groups, for example, trihydroxy or octahydroxy functional products.
For the long chain polyol (e), it is preferable to use a long chain polyol (e) containing at least a trihydroxy functional product. The long chain polyol (e) may be a polyol containing a trifunctional product only, or may be a polyol containing a polyfunctional product other than a trifunctional product. Examples of the other polyfunctional product include a bifunctional product, and tetrafunctional or higher-functional products. Specifically, a mixture of a trifunctional product and a bifunctional product can be used.
The average molecular weight of the long chain polyol (e) can be adjusted to, for example, 200 to 10,000 (preferably, 200 to 5,000). By adjusting the average molecular weight in this range, it is made difficult to achieve gelation, and the handleability at the time of application can be made satisfactory.
In the case of using a mixture of a trifunctional product and another functional product, it is preferable to adjust the ratio of the trifunctional product in the long chain polyol (e) to a molar ration of 50% or greater.

The short chain polyol (d) is a polyol having a molecular weight of less than 200, and a short chain diol, a short chain triol and the like can be used.
Examples of the short chain diol include 1,4-butanediol (molecular weight 90), 3-methyl-1,5-pentadiol (molecular weight 118), and 1,9-nonanediol (molecular weight 160).
Examples of the short chain triol include trimethylolpropane (molecular weight 134), and tripropylene glycol (molecular weight 192).
For the short chain polyol (d), only any one of a short chain diol and a short chain triol may be used, or the two may be used together. Specifically, one or two or more selected from the compounds mentioned above (1,4-butanediol, 3-methyl-1,5-pentadiol, 1,9-nonanediol, trimethylolpropane and tripropylene glycol) can be used.

The ratio of the hydroxyl groups (OH group) of the short chain polyol (d) occupying the polyol component (a) (proportion of the amount of OH groups of the short chain polyol (d) occupying in the total amount of OH groups of the polyol component (a)) (molar ratio) is preferably 40% or less, and for example, 10 to 20% is suitable.
When the ratio of the short chain polyol (d) is adjusted in the range mentioned above, the curing rate is suppressed, and a sufficient usable time can be secured.
Here, the usable time means a time period in which, upon coating a mixed liquid of the main agent (A) and the curing agent (B), the mixed liquid has sufficient fluidity and can be smoothly coated. The usable time can be expressed by taking, for example, the time for the viscosity to reach 30,000 mPa·s as an indicator.

As the MDI, pure MDI, liquid MDI and the like can be used. The liquid MDI is a product obtainable by treating so-called pure MDI using a known catalyst such as a phosphorene-based or an alkyl phosphate-based catalyst, and modifying a portion of the NCO groups. The liquid MDI is liquid at normal temperature.
For example, use can be made of pure MDI in an amount containing NCO groups such that the molar ratio with respect to the OH groups of the polyol component (a) is 1:1, and of a liquid MDI in an amount that constitutes a surplus MDI (c).

The mixing ratio of the polyol component (a) and MDI is set up such that the mole number of the isocyanate groups (NCO groups) of MDI is larger than the mole number of the OH groups of the polyol component (a), that is, there is an excess of MDI.
Specifically, the mixing ratio of the polyol component (a) and MDI can be set up such that the content of NCO groups in the main agent (A) would be (theoretically) 4 to 9% by mass, and preferably 6 to 7% by mass.
The content of NCO groups in the main agent (A) can be calculated as follows. The difference between the amount of NCO groups of MDI and the amount of OH groups of the polyol component (a) added to the main agent (A) (NCO groups - OH groups) (on a molar basis), is designated as the amount (on a molar basis) of NCO groups (NCO groups not bound to OH groups) (free NCO groups), and the mass of NCO groups is obtained by multiplying this amount of NCO groups by the molecular weight of NCO (about 42). Then, the content (on a mass basis) of NCO groups in the main agent (A) can be calculated based on the mass of NCO groups.
If the content of NCO groups is too small, the stickiness of the coating film surface is increased, and if the content is too large, the usable time tends to be shortened. However, when the content is set in the range mentioned above, the stickiness of the coating film surface can be suppressed, and a sufficient usable time can be secured.
The amount of surplus MDI (c) can be decided so as to adjust the content of NCO groups to the range mentioned above.

The main agent (A) can be incorporated with additives such as a solvent, a plasticizer and a defoamer, if necessary.
The solvent functions to regulate properties such as viscosity, and examples include aromatic hydrocarbons such as toluene, xylene and styrene, and chlorination products thereof.
As the plasticizer, carboxylic acid esters such as phthalic acid esters, adipic acid esters, sebacic acid esters, azelaic acid esters, and trimellitic acid esters can be used, and particularly, diisononyl adipate (DINA), di-2-ethylhexyl phthalate (DOP) and the like are preferred.
Examples of the defoamer include dimethylsiloxane-based defoamers and polyacrylate-based defoamers.

### (2) Regarding curing agent (B)

The curing agent (B) contains a crosslinking agent (g) obtained by a short chain polyol (f), and a long chain polyol (h).
The short chain polyol (f) is a polyol having a molecular weight of less than 200, and a short chain diol, a short chain triol and the like can be used.
Examples of the short chain diol include 1,4-butanediol (molecular weight 90), 3-methyl-1,5-pentadiol (molecular weight 118), and 1,9-nonanediol (molecular weight 160).
Examples of the short chain triol include trimethylolpropane (molecular weight 134) and tripropylene glycol (molecular weight 192).
For the short chain polyol (f), only any one of the short chain diol and the short chain triol may be used, or two thereof may be used together. Specifically, one or two or more selected from the compounds mentioned above (1,4-butanediol, 3-methyl-1,5-pentadiol, 1,9-nonanediol, trimethylolpropane and tripropylene glycol) can be used.

The long chain polyol (h) is a polyol having a molecular weight (average molecular weight) of 200 or greater, and for example, one or two or more among a polyether polyol, a polyester polyol and a polycarbonate polyol can be used.
Examples of the polyether polyol include polyethylene glycol, polypropylene glycol and polytetramethylene glycol.
Examples of the polyester polyol include polyethylene adipate diol, polybutylene adipate diol, polyethylene succinate diol, and polybutylene succinate diol.
Examples of the polycarbonate polyol include polytetramethylene carbonate diol, and polyhexamethylene carbonate diol.
Furthermore, a polymeric polyol or a plant-derived polyol (castor oil, linseed oil or the like) can also be used.

As the long chain polyol (h), these compounds may be used individually, or a mixture of two or more compounds may be used.
As the long chain polyol (h), a bifunctional product may be used, or a trifunctional or higher-functional product may be used. A mixture of these may also be used.

In regard to the mixing ratio of the crosslinking agent (g) (short chain polyol (f)), the ratio of OH groups (proportion of the amount of OH groups of the crosslinking agent (g) occupying the total amount of OH groups of the crosslinking agent (g) and the long chain polyol (h)) (molar ratio) can be adjusted to, for example, 60 to 90%. When the ratio is adjusted to this range, the stickiness of the coating film surface can be suppressed, and handling of the crosslinking agent can be made easy.

A catalyst can be added to the curing agent (B).
As the catalyst, an organometallic catalyst such as lead naphthenate or lead octanoate can be used.
Although a short chain polyol is slow in response to the NCO group of the main agent (A) as compared with amine compounds, the addition of a catalyst promotes the reaction between the OH groups of the short chain polyol (f) and the NCO groups of the main agent (A), and can increase the curing rate.
When the amount of the addition of the catalyst is adjusted to, for example, 0.05 to 5 parts by mass relative to 100 parts by mass of the isocyanate group-terminated prepolymer (b), the curing rate can be increased, and the temperature increase during reaction can be suppressed.

The curing agent (B) can be incorporated with additives such as a solvent, a plasticizer, a defoamer, a filler, a powder, an antioxidant, an ultraviolet absorbent, a precipitation preventing agent, and a pigment, if necessary.
The solvent functions to regulate properties such as viscosity, and examples include aromatic hydrocarbons such as toluene, xylene and styrene, and chlorination products thereof.
As the plasticizer, carboxylic acid esters such as phthalic acid esters, adipic acid esters, sebacic acid esters, azelaic acid esters, and trimellitic acid esters can be used, and particularly, diisononyl adipate (DINA), di-2-ethylhexyl phthalate (DOP) and the like are preferred.
Examples of the defoamer include dimethylsiloxane-based defoamers and polyacrylate-based defoamers.
The filler is added for the purpose of reducing shrinkage upon polymerization, causing a weight increase, enhancing the hardness, or the like, and calcium carbonate, clay, talc, silica, diatomaceous earth and the like can be used.

The mixing ratio of the NCO groups of the main agent (A) and the OH groups of the curing agent (B), the molar ration of NCO group/OH group (the NCO/OH index) is preferably 1.2 to 1.3.
When the NCO/OH index is adjusted to the range mentioned above, the properties of the cured product are improved, and foaming upon mixing of the main agent (A) and the curing agent (B) can be prevented.

Next, the method for producing the urethane resin composition will be described.
According to the present invention, the polyol component (a) is mixed with MDI, and thus an isocyanate group-terminated prepolymer (b) is produced.
The mixing ratio of the polyol component (a) and MDI is adjusted such that the mole number of the isocyanate groups (NCO groups) of MDI is larger than the mole number of the OH groups of the polyol component (a), that is, MDI is in excess.
Specifically, the mixing ratio of the polyol component (a) and MDI can be set up such that the content of NCO groups in the main agent (A) would be (theoretically) 4 to 9% by mass, and preferably 6 to 7% by mass. When the content of NCO groups (NCO%) is adjusted to 4 to 9% by mass, a urethane resin composition is obtained which has improved properties such as surface stickiness and strength, and which does not have a problem of foaming.
Thereby, a main agent (A) containing an isocyanate group-terminated prepolymer (b) having MDI bound to the termini of a polyol component (a), and surplus MDI (c), is obtained.

Since the polyol component (a) contains a short chain polyol (d) and a long chain polyol (e), the isocyanate group-terminated prepolymer (b) becomes a mixture of a plurality of isocyanate group-terminated compounds.
For example, when the short chain polyol (d) contains a short chain diol and a short chain triol, an isocyanate group-terminated compound having MDI respectively bound to two OH groups is produced from the short chain diol, and an isocyanate group-terminated compound having MDI respectively bound to three OH groups is produced from the short chain triol, while an isocyanate group-terminated compound having MDI respectively bound to each OH group of the long chain polyol (e) is produced from the long chain polyol (e).
In this case, the isocyanate group-terminated prepolymer (b) becomes a mixture of a first isocyanate group-terminated compound (derived from short chain diol), a second isocyanate group-terminated compound (derived from short chain triol), and a third isocyanate group-terminated compound (derived from a long chain polyol (e)).
In regard to the main agent (A), since a short chain polyol (d) is used as the polyol component (a), the mechanical strength of the coating film can be increased, and at the same time, the coating film can be greatly stretched.

The curing agent (B) can be prepared by mixing a crosslinking agent (g) obtained by a short chain polyol (f); a long chain polyol (h); and the additives mentioned above (a catalyst and the like).

Next, the main agent (A) and the curing agent (B) are mixed and reacted, and thus a urethane resin composition is produced.
In this urethane resin composition, since a crosslinking agent (g) obtained by a short chain polyol (f) is used in the curing agent (B), the curing rate is suppressed even though MDI is used as the isocyanate component.
For example, the time for the viscosity of the mixed liquid to reach 30,000 mPa·s at 23°C can be adjusted to 10 to 120 minutes. The time to reach 30,000 mPa·s is preferably 30 to 50 minutes.
The tack-free drying time (time taken until the coating film does not undergo deformation even if the worker climbs on the coating film) can be adjusted to, for example, 8 to 36 hours. The tack-free drying time is preferably adjusted to 16 to 24 hours.

The urethane resin composition according to the present can produce a waterproof coating film (urethane waterproofing material) when applied and cured on the rooftops, verandas, corridors and the like of buildings.
Since the curing rate can be suppressed, a sufficient usable time can be secured. Thus, a hand painting application can be achieved by using a coating tool such as a trowel, a spatula, a rake or a squeegee, and the urethane resin composition is excellent as a coating agent for a hand painting application.
Furthermore, since MDI which is less harmful to the human body is used, the urethane resin composition is excellent in terms of safety.
In addition, according to the invention, the hand painting application is not limited to the application using a coating tool such as a trowel, a spatula, a rake or a squeegee. For example, an application using a pressure feeding apparatus having a main agent container for storing the main agent; a curing agent container for storing the curing agent; a mixer for mixing the main agent and curing agent separately transported through pipes from the containers; an ejection unit, is also included in a hand painting application.
In the application using a pressure feeding apparatus, for example, the urethane resin compound according to the present invention can be coated on a surface of a product to be coated by mixing the main agent and the curing agent respectively transported through pipes from the respective containers, at a predetermined ratio in the mixer, and ejecting the mixed liquid from the ejection unit. The ejection unit can also supply the mixed liquid to the surface intended for application, without spraying.

### [EXAMPLES]

Hereinafter, the invention will be described in detail with reference to specific examples. In addition, the "parts" indicating the amount of mixing means "parts by mass."

### <Test Examples 1 to 3>

Urethane resin compositions were prepared as follows.

### (1) Preparation of main agent (A)

A polyol component (a) obtained by 1,4-butanediol (1,4BD manufactured by Mitsui Chemicals Polyurethanes, Inc.) (short chain polyol (d)), polyether polyol (polypropylene glycol) (Diol-2000 manufactured by Mitsui Chemicals Polyurethanes, Inc.: dihydroxy functional product having an average molecular weight of 2,000) (long chain polyol (e)), and a polyether polyol (modified polypropylene glycol) (MN-3050K manufactured by Mitsui Chemicals Polyurethanes, Inc.: trihydroxy functional product having an average molecular weight of 3,000) (long chain polyol (e)) was mixed with pure MDI (Cosmonate PH manufactured by Mitsui Chemicals Polyurethanes, Inc.), liquid MDI (MDI-LL manufactured by Mitsui Chemicals Polyurethanes, Inc., isocyanate content 29.1% by mass), and diisononyl adipate (DINA) (plasticizer), and thus an isocyanate group-terminated prepolymer (b) was produced.
The mixing ratios of the respective components are shown in Table 1.
In the Table 1, the NCO/OH index represents the mixing ratio of the NCO groups of MDI and the OH groups of the polyol component (a) (NCO/OH) (molar ratio).
The NCO% is the mass% of the NCO groups in the main agent (A). Hereinafter, the same indications will be employed for the main agent (A).
In Table 1, pure MDI is indicated as a material constituting the isocyanate group-terminated prepolymer (b), and liquid MDI is indicated as a material constituting the surplus MDI (c). However, a portion of the pure MDI may become the surplus MDI (c), and a portion of the liquid MDI may become the material constituting the isocyanate group-terminated prepolymer (b).

**[Table 1]**

| Main agent (A) | | | | Test Example 1 | Test Example 2 | Test Example 3 |
|---|---|---|---|---|---|---|
| Prepolymer (b) (parts) | Isocyanate component | MDI | Pure MDI | 18.89 | 18.89 | 18.89 |
| | Polyol component (a) | Short chain polyol (d) | 1,4BD | 0.68 | 0.68 | 0.68 |
| | | Long chain polyol (e) | Polyether polyol (bifunctional product) | 60.43 | 30.22 | 0 |
| | | | Polyether polyol (trifunctional product) | 0 | 30.21 | 60.43 |
| Surplus MDI (c) (parts) | | MDI | Liquid MDI | 10 | 10 | 10 |
| Additive (parts) | | Plasticizer | DINA | 10 | 10 | 10 |
| Total (parts) | | | | 100 | 100 | 100 |
| NCO/OH index (-) | | | | 2.9 | 2.9 | 2.9 |
| NCO% | | | | 6.07 | 6.07 | 6.07 |

### (2) Preparation of curing agent (B)

A crosslinking agent (g) obtained by 1,4-butanediol (1,4BD manufactured by Mitsui Chemicals Polyurethanes, Inc.) (short chain polyol (f)), a polymeric polyol (trifunctional product) (KC900 manufactured by Sanyo Chemical Industries, Ltd.) (long chain polyol (h)) (average molecular weight: 5,000), a polyether polyol (bifunctional product) (Actcol ED-37B manufactured by Mitsui Chemicals Polyurethanes, Inc.) (a long chain polyol (h)) (an average molecular weight: 3,000), lead naphthenate (Naphthex Pb-24 manufactured by Nihon Kagaku Sangyo Co., Ltd.) (catalyst (i)), a defoamer (Flowren AC1190 manufactured by Kyoeisha Chemical Co., Ltd.), terpene (solvent), di-2-ethylhexyl phthalate (DOP) (plasticizer), and calcium carbonate (NS200 manufactured by Nitto Funka Kogyo K.K.) (filler) were mixed to obtain a curing agent (B).
The mixing ratios of the respective components are shown in Table 2. In the Table 2, the NCO/OH index represents the mixing ratio of the NCO groups of the main agent (A) and the OH groups of the curing agent (B) (OH groups of the crosslinking agent (g) and the long chain polyol (h)) (NCO/OH) (molar ratio). Hereinafter, the same indication will be employed for the curing agent (B).

**[Table 2]**

| Curing agent (B) | | | Test Examples 1-3 |
|---|---|---|---|
| Crosslinking agent (g) (parts) | Short chain polyol (f) | 1,4BD | 4.34 |
| Long chain polyol (h) (parts) | | Polymeric polyol (trifunctional product) | 31.3 |
| | | Polyether polyol (bifunctional product) | 18.06 |
| Additive (parts) | Catalyst | Lead naphthenate | 1 |
| | Defoamer | | 1 |
| | Solvent | Terpene | 10 |
| | Plasticizer | DOP | 20 |
| | Filler | Calcium carbonate | 114.3 |
| Total (parts) | | | 200 |
| NCO/OH index (-) | | | 1.2 |

The urethane resin composition obtained by mixing the main agent (A) and the curing agent (B) was cured, and the following properties of the cured product were measured. The results are shown in Table 3.
The tensile strength: the tensile strength [N/mm²] as defined in JIS A6021-2000.
The elongation ratio: the elongation ratio upon fracture [%] as defined in JIS A6021-2000.
The rupture strength: the rupture strength [N/mm] as defined in JIS A6021-2000.
Stickiness of surface: the stickiness of the surface obtainable 24 hours after mixing was investigated.
The stickiness of the surface was evaluated to be in any of a state completely free from stickiness (Excellent), a state having slight stickiness remaining behind (Good), a state having stickiness remaining to an extent not causing problems in practical use (Inferiro), and a state having stickiness remaining to an extent causing problems in practical use (Bad).

**[Table 3]**

| Properties | Test Example 1 | Test Example 2 | Test Example 3 |
|---|---|---|---|
| Tensile strength (N/mm²) | 0.631 | 3.74 | 4.84 |
| Elongation ratio (%) | 230 | 410 | 330 |
| Rupture strength (N/mm) | 6.77 | 15.6 | 17.1 |
| Stickiness of surface | Good | Excellent | Excellent |

From Table 3, it is found that since a short chain polyol (f) was used as the crosslinking agent (g) in the curing agent (B), a cured product having excellent mechanical properties such as strength and elongation ratio was obtained, and furthermore, the stickiness of the surface was reduced in a short time.
Furthermore, by using a trifunctional product as the long chain polyol (e), excellent properties were obtained.

### <Test Examples 4 and 5>

Urethane resin compositions were prepared as follows.

### (1) Preparation of main agent (A)

A polyol component (a) obtained by a short chain polyol (d), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL) and diisononyl adipate (DINA) (plasticizer), and thus an isocyanate group-terminated prepolymer (b) was produced.
As the short chain polyol (d), 1,4-butanediol (1,4BD) or 3-methyl-1,5-pentadiol (MPD) was used.
The mixing ratios of the respective components are shown in Table 4.

**[Table 4]**

| Main agent (A) | | | | Test Example 4 | Test Example 5 |
|---|---|---|---|---|---|
| Prepolymer (b) (parts) | Isocyanate component | MDI | Pure MDI | 15.35 | 15.35 |
| | Polyol component (a) | Short chain | 1,4BD | 0.55 | 0 |
| | | polyol (d) | MPD | 0 | 0.72 |
| | | Long chain polyol (e) | Polyether polyol (bifunctional product) | 24.55 | 24.55 |
| | | | Polyether polyol (trifunctional product) | 24.55 | 24.55 |
| Surplus MDI | (c) (parts) | MDI | Liquid MDI | 15 | 15 |
| Additive | (parts) | Plasticizer | DINA | 20 | 19.83 |
| Total (parts) | | | | 100 | 100 |
| Nature (23°C) | | | | Liquid | Liquid |
| NCO/OH index (-) | | | | 3.7 | 3.7 |
| NCO% | | | | 6.92 | 6.92 |

### (2) Preparation of curing agent (B)

A crosslinking agent (g) obtained by 1,4-butanediol (1,4BD) (short chain polyol (f)), a polymeric polyol (trifunctional product) (KC900) (long chain polyol (h)), and a polyether polyol (bifunctional product) (ED-37B) (long chain polyol (h)) were mixed with the same additives (catalyst, defoamer, solvent, plasticizer and filler) as those used in Test Example 1 and talc (PK50 manufactured by Fuji Talc Industrial Co., Ltd.) (filler), and the mixture was used as a curing agent (B).
The mixing ratios of the respective components are shown in Table 5.

**[Table 5]**

| Curing agent (B) | | | Test Examples 4 and 5 |
|---|---|---|---|
| Crosslinking agent (g) (parts) | Short chain polyol (f) | 1,4BD | 4.94 |
| Long chain polyol (h) (parts) | | Polymeric polyol (trifunctional product) | 26.77 |
| | | Polyether polyol (bifunctional product) | 25.74 |
| Additive (parts) | Catalyst | Lead naphthenate | 1 |
| | Defoamer | | 1 |
| | Solvent | Terpene | 10 |
| | Plasticizer | DOP | 16 |
| | Filler | Calcium carbonate | 104.55 |
| | | Talc | 10 |
| Total (parts) | | | 200 |
| NCO/OH index (-) | | | 1.2 |

A urethane resin composition obtained by mixing the main agent (A) and the curing agent (B) was cured, and the results of measuring the properties of the cured product are shown in Table 6.

**[Table 6]**

| Properties | Test Example 4 | Test Example 5 |
|---|---|---|
| Tensile strength (N/mm²) | 4.24 | 4.85 |
| Elongation ratio (%) | 530 | 530 |
| Rupture strength (N/mm) | 17.7 | 18.0 |

It is found from Table 6 that satisfactory properties were obtained even when one or more of 1,4-butanediol and 3-methyl-1,5-pentadiol were used.

### <Test Examples 6 to 12>

Urethane resin compositions were prepared as follows.

### (1) Preparation of main agent (A)

A polyol component (a) obtained by 1,4-butanediol (1,4BD) (short chain polyol (d)), a polyether polyol (bifunctional product) (Diol-2000) (long chain polyol (e)), and a polyether polyol (trifunctional product) (MN-3050K) (long chain polyol (e)) were mixed with pure MDI (Cosmonate PH), liquid MDI (MDI-LL), and diisononyl adipate (DINA) (plasticizer), and thus an isocyanate group-terminated prepolymer (b) was produced.
The mixing ratios of the respective components are shown in Table 7.

**[Table 7]**

| Main agent (A) | | | | Test Examples 6-12 |
|---|---|---|---|---|
| Prepolymer (b) (parts) | Isocyanate component | MDI | Pure MDI | 18.89 |
| | Polyol component (a) | Short chain polyol (d) | 1,4BD | 0.68 |
| | | Long chain polyol (e) | Polyether polyol (bifunctional product) | 30.22 |
| | | | Polyether polyol (trifunctional product) | 30.21 |
| Surplus MDI (c) (parts) | | MDI | Liquid MDI | 10 |
| Additive (parts) | | Plasticizer | DINA | 10 |
| Total (parts) | | | | 100 |
| NCO/OH index (-) | | | | 2.9 |
| NCO% | | | | 6.07 |

### (2) Preparation of curing agent (B)

A crosslinking agent (g) obtained by 1,4-butanediol (1,4BD) (short chain polyol (f)), a polymeric polyol (trifunctional product) (KC900) (long chain polyol (h)), and a polyether polyol (bifunctional product) (ED-37B) (long chain polyol (h)) were mixed with the same additives (catalyst, defoamer, solvent, plasticizer, and filler) as those used in Test Example 1, and the mixture was used as a curing agent (B).
The mixing ratios of the respective components are shown in Table 8. In the Table 8, the "molar ratio in the OH groups of the polyol component" means the ratio of OH groups of the respective components with respect to the total of OH groups of the crosslinking agent (g) and the long chain polyol (h). Hereinafter, the same indication will be employed for the curing agent (B).

**[Table 8]**

| Curing agent (B) | | | Test Example 6 | Test Example 7 | Test Example 8 | Test Example 9 | Test Example 10 | Test Example 11 | Test Example 12 |
|---|---|---|---|---|---|---|---|---|---|
| Crosslinking agent (g) (parts) | Short chain polyol (f) | 1,4BD | 5.15 | 4.88 | 4.34 | 3.25 | 2.71 | 2.17 | 1.63 |
| | | (Molar ratio in OH groups of polyol component (%)) | (95) | (90) | (80) | (60) | (50) | (40) | (30) |
| Long chain polyol (h) (parts) | | Polymeric polyol (trifunctional product) | 15.66 | 31.3 | 31.3 | 31.3 | 31.3 | 31.3 | 31.3 |
| | | (Molar ratio in OH groups of polyol component (%)) | (5) | (10) | (10) | (10) | (10) | (10) | (10) |
| | | Polyether polyol (bifunctional product) | 0 | 0 | 18.06 | 54.18 | 72.26 | 90.3 | 108.4 |
| | | (Molar ratio in OH groups of polyol component (%)) | (0) | (0) | (10) | (30) | (40) | (50) | (60) |
| Additive (parts) | Catalyst | Lead naphthenate | 1 | 1 | 1 | 1 | 1 | 1 | 1 |
| | Defoamer | | 1 | 1 | 1 | 1 | 1 | 1 | 1 |
| | Solvent | Terpene | 10 | 10 | 10 | 10 | 10 | 10 | 10 |
| | Plasticizer | DOP | 20 | 20 | 20 | 20 | 20 | 20 | 20 |
| | Filler | Calcium carbonate | 147.19 | 130.81 | 114.3 | 79.27 | 61.73 | 44.23 | 25.67 |
| Total (parts) | | | 200 | 200 | 200 | 200 | 200 | 200 | 200 |
| NCO/OH index (-) | | | 1.2 | 1.2 | 1.2 | 1.2 | 1.2 | 1.2 | 1.2 |

In the Test Example 6 (molar ratio of OH groups of the short chain polyol (f) 95%), the curing agent (B) acquired a nature close to a solid, and the handling thereof was difficult.
The urethane resin composition obtained by mixing the main agent (A) and the curing agent (B) was cured, and the properties of the cured product were measured. The results are shown in Table 9.

**[Table 9]**

| Properties | Test Example 6 | Test Example 7 | Test Example 8 | Test Example 9 | Test Example 10 | Test Example 11 | Test Example 12 |
|---|---|---|---|---|---|---|---|
| Tensile strength (N/mm²₎ | 5.91 | 4.23 | 3.74 | 3.13 | 1.97 | 2.44 | 1.85 |
| Elongation ratio (%) | 430 | 420 | 410 | 590 | 700 | 710 | 610 |
| Rupture strength (N/mm) | 19.9 | 16.3 | 15.6 | 11.2 | 7.51 | 8.31 | 6.58 |
| Stickiness of surface | Excellent | Excellent | Excellent | Good | Inferior | Inferior | Inferior |

It is found from the Table 9 that as the ratio of the crosslinking agent (g) (short chain polyol (f)) in the curing agent (B) increased, stickiness of the cured product surface or the properties such as strength tended to become satisfactory. Particularly, it is found that when the ratio was adjusted to 60% or higher, the stickiness of the cured product surface decreased, and the stretching and rupture strength also became satisfactory.
Furthermore, in the Test Examples where the ratio of the crosslinking agent (g) (short chain polyol (f)) was 90% or lower, the handleability was satisfactory.
Therefore, it can be seen that by adjusting the ratio to 60 to 90%, a urethane resin composition which has satisfactory stickiness of the surface and properties such as strength and has no problem in terms of handleability, was obtained.

### <Test Examples 13 to 15>

Urethane resin compositions were prepared as follows.

### (1) Preparation of main agent (A)

A polyol component (a) obtained by 1,4-butanediol (1,4BD) (short chain polyol (d)), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL), and diisononyl adipate (DINA) (plasticizer), and thus an isocyanate group-terminated prepolymer (b) was produced.
The mixing ratios of the respective components are shown in Table 1.

**[Table 10]**

| Main agent (A) | | | | Test Examples 13-15 |
|---|---|---|---|---|
| Prepolymer (b) (parts) | Isocyanate component | MDI | Pure MDI | 18.89 |
| | Polyol component (a) | Short chain polyol (d) | 1,4BD | 0.68 |
| | | Long chain polyol (e) | Polyether polyol (bifunctional product) | 30.22 |
| | | | Polyether polyol (trifunctional product) | 30.21 |
| Surplus MDI (c) (parts) | | MDI | Liquid MDI | 10 |
| Additive (parts) | | Plasticizer | DINA | 10 |
| Total (parts) | | | | 100 |
| NCO/OH index (-) | | | | 2.9 |
| NCO% | | | | 6.07 |

### (2) Preparation of curing agent (B)

A crosslinking agent (g) obtained by a short chain polyol (f), a polymeric polyol (trifunctional product) (KC900) (long chain polyol (h)), and a polyether polyol (bifunctional product) (ED-37B) (long chain polyol (h)) were mixed with the same additives (catalyst, defoamer, solvent, plasticizer and filler) as those used in Test Example 1, and the mixture was used as a curing agent (B).
As the short chain polyol (f), one of 1,4-butanediol (1,4BD), 3-methyl-1,5-pentadiol (MPD) and 1,9-nonanediol (1,9-ND) was used.
The mixing ratios of the respective components are shown in Table 11.

**[Table 11]**

| Curing agent (B) | | | Test Example 13 | Test Example 14 | Test Example 15 |
|---|---|---|---|---|---|
| Crosslinking agent (g) (parts) | Short chain polyol (f) | 1,4BD | 4.34 | - | - |
| | | (Molar ratio in OH groups of polyol component (%)) | (80) | - | - |
| | | MPD | - | 5.68 | |
| | | (Molar ratio in OH groups of polyol component (%)) | - | (80) | |
| | | 1,9ND | - | - | 7.61 |
| | | (Molar ratio in OH groups of polyol component (%)) | - | - | (80) |
| Long chain polyol (h) (parts) | | Polymeric polyol (trifunctional product) | 31.3 | 31.3 | 31.3 |
| | | (Molar ratio in OH groups of polyol component (%)) | (10) | (10) | (10) |
| | | Polyether polyol (bifunctional product) | 18.06 | 18.06 | 18.06 |
| | | (Molar ratio in OH groups of polyol component (%)) | (10) | (10) | (10) |
| Additive (parts) | Catalyst | Lead naphthenate | 1 | 1 | 1 |
| | Defoamer | | 1 | 1 | 1 |
| | Solvent | Terpene | 10 | 10 | 10 |
| | Plasticizer | DOP | 20 | 20 | 20 |
| | Filler | Calcium carbonate | 114.3 | 112.96 | 111.03 |
| Total (parts) | | | 200 | 200 | 200 |
| NCO/OH index (-) | | | 1.2 | 1.2 | 1.2 |

The urethane resin composition obtained by mixing the main agent (A) and the curing agent (B) was cured, and the properties of the cured product were measured. The results are shown in Table 12.

**[Table 12]**

| Properties | Test Example 13 | Test Example 14 | Test Example 15 |
|---|---|---|---|
| Tensile strength (N/mm²) | 3.74 | 3.36 | 4.45 |
| Elongation ratio (%) | 410 | 510 | 490 |
| Rupture strength (N/mm) | 15.6 | 11.4 | 14.7 |

It can be seen from the Table 12 that even if any short chain polyol (f) is used in the curing agent (B), the respective properties become satisfactory.

### <Test Examples 16 to 18>

Urethane resin compositions were prepared as follows.

### (1) Preparation of main agent (A)

A polyol component (a) obtained by 1,4-butanediol (1,4BD) (short chain polyol (d)), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL) and diisononyl adipate (DINA) (plasticizer), and thus an isocyanate group-terminated prepolymer (b) was produced.
The mixing ratios of the respective components are shown in Table 13.

**[Table 13]**

| Main agent (A) | | | | Test Examples 16-18 |
|---|---|---|---|---|
| Prepolymer (b) (parts) | Isocyanate component | MDI | Pure MDI | 18.89 |
| | Polyol component (a) | Short chain polyol (d) | 1,4BD | 0.68 |
| | | Long chain | Polyether polyol (bifunctional product) | 30.22 |
| | | polyol (e) | Polyether polyol (trifunctional product) | 30.21 |
| Surplus MDI (c) (parts) | | MDI | Liquid MDI | 10 |
| Additive (parts) | | Plasticizer | DINA | 10 |
| Total (parts) | | | | 100 |
| NCO/OH index (-) | | | | 2.9 |
| NCO% | | | | 6.07 |

### (2) Preparation of curing agent (B)

A crosslinking agent (g) obtained by 1,4-butanediol (1,4BD) (short chain polyol (f)), and a long chain polyol (h) were mixed with the same additives (catalyst, defoamer, solvent, plasticizer and filler) as those used in Test Example 1, and the mixture was used as a curing agent (B).

As the long chain polyol (h), a mixture of any one of a polymeric polyol (trifunctional product) (KC900), a polyether polyol (MN-5000 manufactured by Mitsui Chemicals Polyurethanes, Inc.: a trihydroxy functional product having an average molecular weight of 5,000), and a castor oil-modified polyol (H1824 manufactured by Itoh Oil Chemicals Co., Ltd.: a mixture of dihydroxy functional product and trihydroxy functional product) (average molecular weight: 1,900), with a polyether polyol (bifunctional product) (ED-37B) was used.
The mixing ratios of the respective components are shown in Table 14.

**[Table 14]**

| Curing agent (B) | | | Test Example 16 | Test Example 17 | Test Example 18 |
|---|---|---|---|---|---|
| Crosslinking agent (g) (parts) | Short chain polyol (f) | 1,4BD | 4.34 | 4.34 | 4.34 |
| Long chain polyol (h) (parts) | | Polymeric polyol (trifunctional product) | 31.3 | - | - |
| | | Polyether polyol (trifunctional product) | - | 20.96 | - |
| | | Castor oil-modified polyol (bifunctional/ trifunctional mixture) | - | - | 9.93 |
| | | Polyether polyol (bifunctional product) | 18.06 | 18.06 | 18.06 |
| Additive (parts) | Catalyst | Lead naphthenate | 1 | 1 | 1 |
| | Defoamer | | 1 | 1 | 1 |
| | Solvent | Terpene | 10 | 10 | 10 |
| | Plasticizer | DOP | 20 | 20 | 20 |
| | Filler | Calcium carbonate | 114.3 | 125.54 | 135.67 |
| Total (parts) | | | 200 | 200 | 200 |
| NCO/OH index (-) | | | 1.2 | 1.2 | 1.2 |

The urethane resin composition obtained by mixing the main agent (A) and the curing agent (B) was cured, and the properties of the cured product were measured. The results are shown in Table 15.

**[Table 15]**

| Properties | Test Example 16 | Test Example 17 | Test Example 18 |
|---|---|---|---|
| Tensile strength (N/mm²) | 3.74 | 2.85 | 2.06 |
| Elongation ratio (%) | 410 | 530 | 423 |
| Rupture strength (N/mm) | 15.6 | 15.6 | 14.1 |

It is found from Table 15 that even when a polymeric polyol or castor oil was used as the long chain polyol (h) of the curing agent (B), the respective properties were improved.

### <Test Examples 19 to 21>

Urethane resin compositions were prepared as follows.

### (1) Preparation of main agent (A)

A polyol component (a) obtained by 1,4-butanediol (1,4BD) (short chain polyol (d)), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL), and diisononyl adipate (DINA) (plasticizer), and thus an isocyanate group-terminated prepolymer (b) was produced.
The mixing ratios of the respective components are shown in Table 16.

**[Table 161**

| Main agent (A) | | | | Test Examples 19-21 |
|---|---|---|---|---|
| Prepolymer (b) (parts) | Isocyanate component | MDI | Pure MDI | 18.89 |
| | Polyol component (a) | Short chain polyol (d) | 1,4BD | 0.68 |
| | | Long chain polyol (e) | Polyether polyol (bifunctional product) | 30.22 |
| | | | Polyether polyol (trifunctional product) | 30.21 |
| Surplus MDI (c) (parts) | | MDI | Liquid MDI | 10 |
| Additive (parts) | | Plasticizer | DINA | 10 |
| Total (parts) | | | | 100 |
| NCO/OH index (-) | | | | 2.9 |
| NCO% | | | | 6.07 |

### (2) Preparation of curing agent (B)

A crosslinking agent (g) obtained by 1,4-butanediol (1,4BD) (short chain polyol (f)), a polymeric polyol (trifunctional product) (KC900) (long chain polyol (h)), and a polyether polyol (bifunctional product) (ED-37B) (long chain polyol (h)) were mixed with the same additives (catalyst, defoamer, solvent, plasticizer and filler) as those used in Test Example 1, and the mixture was used as a curing agent (B).
The mixing ratios of the respective components are shown in Table 17.

**[Table 17]**

| Curing agent (B) | | | Test Example 19 | Test Example 20 | Test Example 21 |
|---|---|---|---|---|---|
| Crosslinkin g agent (g) (parts) | Short chain polyol (f) | 1,4BD | 5.79 | 4.34 | 4.01 |
| Long chain polyol (h) (parts) | | Polymeric polyol (trifunctional product) | 41.73 | 31.3 | 28.89 |
| | | Polyether polyol (bifunctional product) | 24.08 | 18.06 | 16.67 |
| Additive (parts) | Catalyst | Lead naphthenate | 1.33 | 1 | 0.92 |
| | Defoamer | | 1.33 | 1 | 0.92 |
| | Solvent | Terpene | 13.33 | 10 | 9.23 |
| | Plasticizer | DOP | 26.67 | 20 | 18.46 |
| | Filler | Calcium carbonate | 152.76 | 114.3 | 105.51 |
| Total (parts) | | | 266.66 | 200 | 184.61 |
| NCO/OH index (-) | | | 0.9 | 1.2 | 1.3 |

The urethane resin composition obtained by mixing the main agent (A) and the curing agent (B) was cured, and the properties of the cured product were measured. The results are shown in Table 18.
It was confirmed that when the mixing ratio of the NCO groups of the main agent (A) and the OH groups of the curing agent (B), NCO group/OH group (molar ratio) (NCO/OH index) exceeded 1.3, foaming was prone to occur upon mixing the main agent (A) and the curing agent (B).

**[Table 18]**

| Properties | Test Example 19 | Test Example 20 | Test Example 21 |
|---|---|---|---|
| Tensile strength (N/mm²) | 0.39 | 3.74 | 4.43 |
| Elongation ratio (%) | 370 | 410 | 410 |
| Rupture strength (N/mm) | 3.87 | 15.6 | 19.1 |

It can be seen from Table 18 that the NCO/OH index is preferably 1.2 to 1.3.

### <Test Example 22>

A urethane resin composition was prepared as follows.

### (1) Preparation of main agent (A)

A polyol component (a) obtained by 1,4-butanediol (1,4BD) (short chain polyol (d)), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL), and diisononyl adipate (DINA) (plasticizer), and thus an isocyanate group-terminated prepolymer (b) was produced.
The mixing ratios of the respective components are shown in Table 19.

**[Table 19]**

| Main agent (A) | | | | Test Example 22 | Test Example 23 |
|---|---|---|---|---|---|
| Prepolymer (b) (parts) | Isocyanate component | MDI | Pure MDI | 18.89 | 29.86 |
| | Polyol component (a) | Short chain polyol (d) | 1,4BD | 0.68 | - |
| | | Long chain polyol (e) | Polyether polyol (bifunctional product) | 30.22 | 48.26 |
| | | | Polyether polyol (trifunctional product) | 30.21 | - |
| plus MDI (c) (parts) | | MDI | Liquid MDI | 10 | 8.92 |
| Additive (parts) | | Plasticizer | DINA | 10 | 12.96 |
| Total (parts) | | | | 100 | 100 |
| NCO% | | | | 6.07 | 10.6 |

### (2) Preparation of curing agent (B)

A crosslinking agent (g) obtained by 1,4-butanediol (1,4BD) (short chain polyol (f)), a polymeric polyol (trifunctional product) (KC900) (long chain polyol (h)), a polyether polyol (bifunctional product) (ED-37B) (long chain polyol (h)), and a polyether polyol (bifuntional product) (Diol-2000) (long chain polyol(h)) were mixed with the same additives (catalyst, defoamer, solvent, plasticizer and filler) as those used in Test Example 1, and the mixture was used as a curing agent (B).
The mixing ratios of the respective components are shown in Table 20.

**[Table 20]**

| Curing agent (B) | | | Test Example 22 | Test Example 23 |
|---|---|---|---|---|
| Crosslinking agent (g) (parts) | diamine | DETDA | - | 15.29 |
| | Short chain polyol (f) | 1,4BD | 4.33 | |
| ng chain polyol (h) (parts) | | Polymeric polyol (KC900) (trifunctional product) | 23.48 | - |
| | | olyether polyol (Diol-2000) (bifunctional product) | - | 28.71 |
| | | Polyether polyol (ED-37B) (bifunctional product) | 22.58 | 43.47 |
| Additive (parts) | Catalyst | Lead naphthenate | 0.5 | 1.45 |
| | Defoamer | | 1 | 0.48 |
| | Solvent | Terpene | 10 | - |
| | Plasticizer | DOP | 20 | 10.6 |
| | Filler | Calcium carbonate | 118.11 | - |
| Total (parts) | | | 200 | 100 |
| NCO/OH index (-) | | | 0.9 | 1.2 |

### <Test Example 23>

The main agent (A) was prepared using DETDA (diethyltoluenediamine) as the crosslinking agent, instead of 1,4-butanediol (1,4BD) (short chain polyol (f)).
The other conditions conformed to Test Example 22.

The urethane resin composition obtained by mixing the main agent (A) and the curing agent (B) was cured. The time taken by the viscosity of the mixed liquid at 23°C to reach 30,000 mPa·s (30,000 mPa·s reaching time) and the tack-free drying time (time taken until the cured product does not undergo deformation even if the worker climbs on the cured product) are shown in Table 21.
In Test Example 23, since the rate of viscosity increase was very high and curing occurred within only several tens of seconds, accurate measurement of the 30000 mPa·s reaching time could not be accomplished, but it was confirmed that the composition was cured in a shorter time as compared with Test Example 22.

**[Table 21]**

| Properties | Test Example 22 | Test Example 23 |
|---|---|---|
| 30,000 mPa·s reaching time | 40 minutes | - |
| ack-free drying time | 16 to 24 hours | 10 minutes |

It is found from Table 21 that as compared with the case of using DETDA as the crosslinking agent, in the Test Examples using a short chain polyol (f) as the crosslinking agent (g), the rate of viscosity increase was suppressed, and the tack-free drying time could be lengthened.

### <Test Examples 24 to 29>

Urethane resin compositions were prepared as follows.

### (1) Preparation of main agent (A)

A polyol component (a) obtained by 1,4-butanediol (1,4BD) (short chain polyol (d)), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL), and diisononyl adipate (DINA) (plasticizer), and thus an isocyanate group-terminated prepolymer (b) was produced.
The mixing ratios of the respective components are shown in Table 22.

**[Table 22]**

| Main agent (A) | | | | Test Example 24 | Test Example 25 | Test Example 26 | Test Example 27 | Test Example 28 | Test Example 29 |
|---|---|---|---|---|---|---|---|---|---|
| Prepolymer (b) (parts) | Isocyanate component | MDI | Pure MDI | 18.89 | 18.89 | 18.89 | 18.89 | 18.89 | 16.53 |
| | Polyol component (a) | Short chain polyol (d) | 1,4BD | 0.68 | 0.68 | 0.68 | 0.68 | 0.68 | 0.6 |
| | | Long chain polyol (e) | Polyether polyol (bifunctional product) | 30.22 | 30.22 | 30.22 | 30.22 | 30.22 | 26.44 |
| | | | Polyether polyol (trifunctional product) | 30.21 | 30.21 | 30.21 | 30.21 | 30.21 | 26.43 |
| Surplus MDI (c) (parts) | | MDI | Liquid MDI | - | 3 | 6.6 | 10 | 20 | 30 |
| Additive (parts) | | Plasticizer | DINA | 20 | 17 | 13.4 | 10 | - | - |
| Total (parts) | | | | 100 | 100 | 100 | 100 | 100 | 100 |
| NCO/OH index (-) | | | | 2.0 | 2.3 | 2.6 | 2.9 | 3.8 | 5.1 |
| NCO% | | | | 3.17 | 4.04 | 5.08 | 6.07 | 8.96 | 11.46 |
| Curing agent (B) Mixing amount (parts) | | | | 104.45 | 133.11 | 167.38 | 200 | 295.22 | 377.59 |

### (2) Preparation of curing agent (B)

A crosslinking agent (g) obtained by 1,4-butanediol (1,4BD) (short chain polyol (f)), a polymeric polyol (trifunctional product) (KC900) (long chain polyol (h)), and a polyether polyol (bifunctional product) (ED-37B) (long chain polyol (h)) were mixed with the same additives (catalyst, defoamer, solvent, plasticizer and filler) as those used in Test Example 1, and the mixture was used as a curing agent (B).
The mixing ratios of the respective components are shown in Table 23.

**[Table 23]**

| Curing agent (B) | | | Test Examples 24-29 |
|---|---|---|---|
| Crosslinking agent (g) (parts) | Short chain polyol (f) | 1,4BD | 4.34 |
| Long chain polyol (h) (parts) | | Polymeric polyol (trifunctional product) | 31.3 |
| | | Polyether polyol (bifunctional product) | 18.06 |
| Additive (parts) | Catalyst | Lead naphthenate | 1 |
| | Defoamer | | 1 |
| | Solvent | Terpene | 10 |
| | Plasticizer | DOP | 20 |
| | Filler | Calcium carbonate | 114.3 |
| Total (parts) | | | 200 |
| NCO/OH index (-) | | | 1.2 |

The urethane resin composition obtained by mixing the main agent (A) and the curing agent (B) was cured, and the properties of the cured product were measured. The results are shown in Table 24.
In the Test Example 29, it was confirmed that foaming was prone to occur upon mixing of the main agent (A) and the curing agent (B).

**[Table 24]**

| Properties | Test Example 24 | Test Example 25 | Test Example 26 | Test Example 27 | Test Example 28 | Test Example 29 |
|---|---|---|---|---|---|---|
| Tensile strength (N/mm²) | 2.75 | 2.72 | - | 3.74 | 4.43 | 4.4 |
| Elongation ratio (%) | 820 | 700 | - | 410 | 470 | 410 |
| Rupture strength (N/mm) | 9.42 | 10.7 | - | 15.6 | 17.6 | 18.1 |
| Stickiness of surface | Inferior | Good | Excellent | Excellent | Excellent | Excellent |

It can be seen from the Table 24 that the NCO% of the main agent (A) was particularly 4% by mass or more, and thus stickiness of the cured product surface was decreased, while stretching and rupture strength also improved.
When the NCO% was 9% by mass or less, the problem of foaming did not occur.

Therefore, it can be seen that when the NCO% of the main agent (A) is adjusted to 4 to 9% by mass, a urethane resin composition was obtained which had a satisfactory surface stickiness and properties such as strength and did not have a problem of foaming.

### <Test Examples 30 to 32>

Urethane resin compositions were prepared as follows.

### (1) Preparation of main agent (A)

A polyol component (a) obtained by a short chain polyol (d), a polyether polyol (bifunctional product) (Diol-2000) (long chain polyol (e)), and a polyether polyol (trifunctional product) (MN-3050K) (long chain polyol (e)) was mixed with pure MDI (Cosmonate PH), liquid MDI (MDI-LL), and diisononyl adipate (DINA) (plasticizer), and thus an isocyanate group-terminated prepolymer (b) was produced.
As the short chain polyol (d), any one of 1,4-butanediol (1,4BD) and tripropylene glycol (TPG), or both were used.
The mixing ratios of the respective components are shown in Table 25.

**[Table 25]**

| Main agent (A) | | | | Test Example 30 | Test Example 31 | Test Example 32 |
|---|---|---|---|---|---|---|
| Prepolymer (b) (parts) | Isocyanate component | MDI | Pure MDI | 18.89 | 18.89 | 18.89 |
| | Polyol component (a) | Short chain polyol (d) | 1,4BD | 0.68 | - | 0.34 |
| | | | TPG | - | 0.97 | 0.49 |
| | | Long chain polyol (e) | Polyether polyol (bifunctional product) | 30.22 | 30.22 | 30.22 |
| | | | Polyether polyol (trifunctional product) | 30.21 | 30.21 | 30.21 |
| Surplus MDI (c) (parts) | MDI | Liquid MDI | 10 | 10 | 10 | |
| Additive (parts) | Plasticizer | DINA | 10 | 9.71 | 9.85 | |
| Total (parts) | | | 100 | 100 | 100 | |
| NCO/OH index (-) | | | 2.9 | 2.9 | 2.9 | |
| NCO% | | | 6.07 | 6.07 | 6.07 | |

### (2) Preparation of curing agent (B)

A crosslinking agent (g) obtained by 1,4-butanediol (1,4BD) (short chain polyol (f)), a polymeric polyol (trifunctional product) (KC900) (long chain polyol (h)), and a polyether polyol (bifunctional product) (ED-37B) (long chain polyol (h)) were mixed with the same additives (catalyst, defoamer, solvent, plasticizer and filler) as those used in Test Example 1, and the mixture was used as a curing agent (B).
The mixing ratios of the respective components are shown in Table 26.

**[Table 26]**

| Curing agent (B) | | | Test Examples 30-32 |
|---|---|---|---|
| Crosslinking agent (g) (parts) | Short chain polyol (f) | 1,4BD | 4.34 |
| Long chain polyol (h) (parts) | | Polymeric polyol (trifunctional product) | 31.3 |
| | | Polyether polyol (bifunctional product) | 18.06 |
| Additive (parts) | Catalyst | Lead naphthenate | 1 |
| | Defoamer | | 1 |
| | Solvent | Terpene | 10 |
| | Plasticizer | DOP | 20 |
| | Filler | Calcium carbonate | 114.3 |
| Total (parts) | | | 200 |
| NCO/OH index (-) | | | 1.2 |

The urethane resin composition obtained by mixing the main agent (A) and the curing agent (B) was cured, and the results of measuring the properties of the cured product are shown in Table 27.

**[Table 27]**

| Properties | Test Example 30 | Test Example 31 | Test Example 32 |
|---|---|---|---|
| Tensile strength (N/mm²) | 3.74 | 3.94 | 4.00 |
| Elongation ratio (%) | 410 | 500 | 550 |
| Rupture strength (N/mm) | 15.6 | 14.0 | 14.7 |

It can be seen from Table 27 that good properties are obtained irrespective of the type of the short chain polyol (d).

## Claims

1. A coating agent for hand painting application comprising a urethane resin composition obtainable by reacting the following main agent (A) and curing agent (B), wherein
the main agent (A) containing:
an isocyanate group-terminated prepolymer (b) having diphenylmethane diisocyanate bound to the termini of a polyol component (a) containing a long chain polyol (e) which contains at least a polyhydroxy functional product such as a trihydroxy or higher-hydroxy functional product and has a molecular weight of 200 or greater, and
unbound diphenylmethane diisocyanate (c); and wherein
the curing agent (B) containing:
a crosslinking agent (g) obtained by a short chain polyol (f) having a molecular weight of less than 200, and
a long chain polyol (h) having a molecular weight of 200 or greater.

2. The coating agent for hand painting application according to claim 1, wherein the mixing ratio of the OH groups of the crosslinking agent (g) with respect to the total amount of the OH groups of the crosslinking agent (g) and the long chain polyol (h) is 60 to 90% on a molar basis in the curing agent (B).

3. The coating agent for hand painting application according to claim 1 or 2, wherein the diphenylmethane diisocyanate and the polyol component (a) are mixed so as to obtain a content ratio of NCO groups of 4 to 9% by mass in the main agent (A).

4. The coating agent for hand painting application according to one of claims 1 to 3, wherein the polyol component (a) in the main agent (A) contains a short chain polyol (d) having a molecular weight of less than 200.

5. The coating agent for hand painting application according to claim 4, wherein the long chain polyol (e) in the main agent (A) is a mixture of a dihydroxy functional product and a trihydroxy functional product.

6. The coating agent for hand painting application according to one of claims 1 to 5, wherein the long chain polyol (h) in the curing agent (B) is a mixture of either a polymeric polyol or a castor oil polyol and a polyether polyol.

7. The coating agent for hand painting application according to one of claims 1 to 5, wherein the long chain polyol (h) in the curing agent (B) is a polyether polyol.

8. A main agent (A) of the coating agent for hand painting application according to claim 1, comprising:
an isocyanate group-terminated prepolymer (b) having diphenylmethane diisocyanate bound to the termini of a polyol component (a) containing a long chain polyol (e) which contains at least a polyhydroxy functional product such as a trihydroxy or higher-hydroxy functional product and has a molecular weight of 200 or greater; and
unbound diphenylmethane diisocyanate (c).

9. A curing agent (B) of the coating agent for hand painting application according to claim 1, comprising:
a crosslinking agent (g) obtained by a short chain polyol (f) having a molecular weight of less than 200, and
a long chain polyol (h) having a molecular weight of 200 or greater.
